# EUROPÄISCHE PATENTSCHRIFT

(11) **EP 1 143 865 B1**
(45) Veröffentlichungstag und Bekanntmachung des Hinweises auf die Patenterteilung: **30.03.2005**
(21) Anmeldenummer: 00909074.7
(22) Anmeldetag: 17.01.2000
(51) Int. Cl.: A61B 17/34, A61B 19/00

(54) **VORRICHTUNG ZUM HALTEN EINER TROKARHÜLSE IN UNTERSCHIEDLICHEN RÄUMLICHEN AUSRICHTUNGEN**
DEVICE FOR HOLDING A TROCAR SLEEVE IN DIFFERENT SPATIAL POSITIONS
DISPOSITIF DE MAINTIEN D'UNE DOUILLE DE TROCART DANS DIFFERENTES POSITIONS SPATIALES

(30) Priorität: 20.01.1999 DE 19902036
(43) Veröffentlichungstag der Anmeldung: 17.10.2001
(73) Patentinhaber: Karl Storz GmbH & Co. KG, 78532 Tuttlingen (DE)
(72) Erfinder: DORN, Jürgen, D-68809 Neulussheim (DE)
(74) Vertreter: Weller, Wolfgang, Dr.
(86) Internationale Anmeldenummer: PCT/EP2000/000309
(87) Internationale Veröffentlichungsnummer: WO 2000/042927

(56) Entgegenhaltungen:
- EP-A- 0 176 452
- WO-A-97/40764
- US-A- 5 284 130
- US-A- 5 540 648
- US-A- 5 540 675

## Beschreibung

Die vorliegende Erfindung betrifft eine Vorrichtung zum Halten einer Trokarhülse in unterschiedlichen räumlichen Ausrichtungen relativ zu einem Patienten, mit einem Basisteil, das relativ zu dem Patienten ortsfest positionierbar ist, und mit einem Haltemittel für die Trokarhülse, wobei das Haltemittel mit dem Basisteil durch ein flexibles Verbindungsglied verkippbar verbunden ist, wobei das Verbindungsglied einen ersten Betriebszustand aufweist, in dem es flexibel ist, sowie einen zweiten Betriebszustand, in dem es steif ist, und wobei das Verbindungsglied zwischen dem ersten flexiblen und dem zweiten steifen Betriebszustand umschaltbar ist.

Eine derartige Haltevorrichtung ist aus der US-A-5,284,130 bekannt.

Ein Trokar dient dazu, bei der minimal-invasiven Chirurgie einen Zugang im Körper zu schaffen. Mit Hilfe eines Trokardorns ist es möglich, eine kleine Inzision anzubringen, über die die Trokarhülse in den Körper des Patienten eingesetzt wird. Im Anschluß daran wird der Trokardorn aus der Trokarhülse herausgezogen. Die Trokarhülse bildet damit einen Zugangskanal in den Körper des Patienten.

Trokare werden vor allem bei minimal-invasiven Eingriffen im Bauchbereich, der Laparoskopie, verwendet. Ihre Verwendung ist jedoch nicht allein hierauf beschränkt. In dem von der Trokarhülse gebildeten Kanal können einerseits medizinische Instrumente sicher geführt oder gehalten werden, und andererseits dient der Kanal auch zum Zuführen oder Absaugen von Flüssigkeiten oder Gasen in bzw. aus dem Körper des Patienten. Dazu ist am proximalen Ende der Trokarhülse üblicherweise ein Klappenventilgehäuse mit entsprechenden Dichthülsen und Sperrhähnen vorgesehen.

Die Trokarhülse steht in einer räumlichen Ausrichtung, die dem Einstichkanal entspricht. Die Trokarhülse kann zwar von Hand in ihrer räumlichen Ausrichtung verändert werden; wird die Trokarhülse freigegeben, richtet sie sich wieder in Richtung des Einstichkanals aus. Dies erfolgt aufgrund der Neigung der vom Trokar durchbohrten Gewebeschichten sich wieder in eine bestimmte Lage zueinander auszurichten.

Aus diesem Grund werden Vorrichtungen der eingangs genannten Art verwendet, um die Trokarhülse in bestimmten Positionen zu halten.

Während einer Operation werden eine oder mehrere unterschiedliche räumliche Ausrichtungen der Trokarhülse gewünscht. In einem ersten Betriebszustand ist daher die Haltevorrichtung, die die Trokarhülse hält, leicht verstellbar. Ist die Trokarhülse in der gewünschten Position ausgerichtet, soll sie dann ihre Lage nicht mehr verändern. In einem zweiten Betriebszustand ist dazu die Haltevorrichtung steif, und die Trokarhülse bleibt in der gewählten räumlichen Ausrichtung fixiert.

Aus der US-A-5,540,675 ist eine Haltevorrichtung bekannt, die ein ringförmiges Basisteil aufweist, das mit Hilfe einer Klebeschicht direkt auf dem Körper des Patienten befestigt wird. Das ringförmige Basisteil ist über elastische Verbindungsglieder aus Gummi mit einem zentrisch zu ihm angeordneten und im Ausgangszustand senkrecht zu ihm stehenden Haltemittel verbunden, das im vorliegenden Fall selbst hülsenförmig ist. In das Haltemittel ist die Trokarhülse eingesteckt. Um das Haltemittel gegenüber dem Basisteil in verschiedenen Richtungen auszurichten und zu fixieren, ist ein teleskopartiger Spannmechanismus vorgesehen, der aus wenigstens einem, in seiner Länge verstellbaren Spannelement besteht. Dieses ist zwischen dem radial außenliegenden Bereich des Basisteils und dem vom Basisteil entfernt liegenden Ende des Haltemittels befestigt. Durch Einstellen der Länge des Spannelements kann dann die Neigung des Haltemittels relativ zu dem Basisteil verändert werden. Hierzu ist es jedoch notwendig, das Spannelement zunächst mittels einer Schraube zu lösen, dann das Spannelement auf die gewünschte Länge einzustellen und anschließend die Schraube wieder festzuziehen. In einem Ausführungsbeispiel dieser bekannten Haltevorrichtung sind aus Stabilitätsgründen drei Spannelemente in Form eines Dreibeins rund um das Haltemittel angeordnet. Mit nur einem Spannelement könnte die Trokarhülse nur in einer einzigen Ebene verkippt werden.

Bei dieser bekannten Haltevorrichtung ist die Einstellung und Fixierung der gewünschten Position des Haltemittels aufwendig und kompliziert. Für eine Änderung der Raumlage der Trokarhülse müssen alle drei Spannelemente zum Fixieren des Haltemittels zunächst gelöst, dann eingestellt und anschließend festgezogen werden. Stellt der Operateur nach Einbringen eines Instruments, bspw. eines Endoskops, fest, daß die Ausrichtung noch nicht exakt ist, muß der komplizierte Vorgang erneut durchgeführt werden. Aufgrund der Dreibeingeometrie sind extreme Kippstellungen der Trokarhülse nicht raumstabil zu erzielen.

Darüber hinaus weisen die Spannelemente dieser bekannten Vorrichtung zahlreiche Bakteriennischen, Hohlräume und Kanten auf, wie beispielsweise die Gewindegänge der Feststellschrauben und die Teleskopschäfte, so daß diese bekannte Vorrichtung schwierig zu reinigen und zu sterilisieren ist.

Bei der Vorrichtung gemäß der eingangs genannten US-A-5,284,130 besteht das flexible Verbindungsglied aus einer Vielzahl von übereinandergestapelten Segmenten, die eine Säule bilden. In dem ersten Betriebszustand können die einzelnen Segmente gegeneinander verdreht und verkippt werden, wodurch sich die Säule insgesamt in verschiedene Richtungen neigen läßt. Über einen Spannmechanismus, der mittig durch die Säule verläuft, können die einzelnen Segmente anschließend gegeneinander verspannt werden, wobei an den Berührungsstellen der Segmente Reibungskräfte die erforderliche Stabilität liefern. Im verspannten, zweiten Betriebszustand ist die Säule steif und behält die eingestellte Neigung bei.

Aus der EP-A-0 176 452 ist eine weitere Vorrichtung zum Halten und Ausrichten eines medizinischen Instruments bekannt. Bei dieser Vorrichtung besitzt das flexible Verbindungsglied eine elastische Hülle, die mit einem partikelförmigen Material gefüllt ist. Im ersten Betriebszustand kann die Hülle mit dem partikelförmigen Material in eine bestimme Ausrichtung verformt werden. Durch Erzeugen eines Unterdrucks in der Hülle kann diese sodann entsprechend dem zweiten Betriebszustand versteift werden.

Es ist eine Aufgabe der vorliegenden Erfindung, eine Vorrichtung der eingangs genannten Art mit einer alternativen Konstruktion für das Verbindungsglied anzugeben, die das Reinigen und Sterilisieren der Vorrichtung nach Gebrauch erleichtert.

Diese Aufgabe wird durch eine Vorrichtung gemäß Anspruch 1 gelöst, bei der das Verbindungsglied eine elastische Hülle aufweist, die mit einem Material veränderbarer Steifigkeit gefüllt ist. Bevorzugte Ausführungen sind in den untergeordneten Ansprüche beschrieben.

Die Vorrichtung kommt ohne teleskopartige oder sonstige mit Schrauben gehaltene Spannelemente aus, deren Bedienung aufwendig und kompliziert ist. An deren Stelle ist demgegenüber erfindungsgemäß vorgesehen, den Grad der Flexibilität bzw. der Steifigkeit des flexiblen Verbindungsglieds selbst variabel zu gestalten. In einem ersten Betriebszustand, in dem das Verbindungsglied flexibel ist, ist es somit möglich, die Trokarhülse und somit auch das darin aufgenommene medizinische Instrument in der gewünschten Position auszurichten. Durch Umschalten in den zweiten Betriebszustand, in dem das Verbindungsglied steif ist, wird die Trokarhülse dann in der eingestellten Position fixiert.

Die Vorrichtung besitzt den Vorteil, daß die Einstellung und die Fixierung der Position der Trokarhülse, und damit des durch diese geführten medizinischen Instruments, in dem ersten flexiblen Betriebszustand sehr einfach in beliebige, auch extreme Kippstellungen möglich ist, während die so eingestellte Position in dem zweiten steifen Betriebszustand sicher und zuverlässig gehalten wird. Die Vorrichtung ist somit gegenüber der gattungsgemäßen Vorrichtung sehr viel einfacher zu bedienen. Gleichzeitig ist es weiterhin möglich, die Vorrichtung mit ihrem Basisteil in unmittelbarer Nähe zu dem zu behandelnden Patienten, beispielsweise auf seine Bauchdecke, aufzusetzen. Dies besitzt den Vorteil, daß die Vorrichtung und somit auch die von ihr gehaltene Trokarhülse Lageveränderungen des Patienten mitmacht. Eine unerwünschte Relativbewegung der Trokarhülse in bezug zu dem Patienten wird vermieden. Schließlich benötigt die Vorrichtung auch nur sehr wenig Platz, so daß ein Zugang zum Patienten von verschiedenen Seiten nicht behindert ist.

Die Vorrichtung besitzt des weiteren den Vorteil, daß das Material in dem ersten Betriebszustand die Eigenschaft einer fließfähigen Masse aufweisen kann, da das Material von der elastischen Hülle zusammengehalten wird. Hierdurch ist ein besonders leichtes Einstellen einer bestimmten Raumausrichtung der Trokarhülse möglich.

Darüber hinaus besitzt die Vorrichtung den Vorteil, daß sie aufgrund der elastischen Hülle, die vorzugsweise aus Silikon ist, eine gleichmäßige und glatte Oberfläche erhält, die einfach zu reinigen und zu sterilisieren ist. Im zweiten Betriebszustand hält die jetzt steife Masse die Trokarhülse raumfest.

In einer Ausgestaltung ist das Material mit einstellbarer Steifigkeit eine Flüssigkeit, deren Viskosität zwischen weichelastisch und steif einstellbar ist.

Derartige Materialien sind im Stand der Technik an sich bekannt. Sie besitzen eine molekulare Struktur, deren Moleküle in verschiedenen Betriebszuständen unterschiedlich fest miteinander verkoppelt und/oder ausgerichtet sind. Dies führt dazu, daß die Flüssigkeiten in den verschiedenen Betriebszuständen stark unterschiedliche Viskositäten aufweisen. Die genannte Maßnahme besitzt den Vorteil, daß auch hierdurch eine sehr einfache und schnell reagierende Möglichkeit gegeben ist, ein erfindungsgemäßes Verbindungsglied mit den gewünschten Betriebszuständen zu realisieren.

In einer weiteren Ausgestaltung der zuvor genannten Maßnahme ist die Viskosität des Materials unter dem Einfluß eines elektrischen und/oder eines magnetischen Feldes einstellbar. Es handelt sich hierbei somit um elektro- oder magnetoviskose Flüssigkeiten, deren Molekularstruktur unter dem Einfluß der genannten Felder veränderbar ist. Alternativ hierzu sind beispielsweise Flüssigkeiten bekannt, deren Viskosität in Abhängigkeit von unterschiedlichen Drücken veränderlich ist. Die genannte Maßnahme besitzt den Vorteil, daß das Umschalten zwischen den beiden erfindungsgemäßen Betriebszuständen sehr einfach und "praktisch auf Knopfdruck" oder bei Bedarf sogar vollständig automatisiert möglich ist.

In einer weiteren Ausgestaltung der eingangs beschriebenen Vorrichtung weist das Verbindungsglied ein Material auf, das bei einer einstellbaren ersten Betriebstemperatur flexibel und bei einer zweiten Betriebstemperatur steif ist. Vorzugsweise ist die zweite Betriebstemperatur die Raumtemperatur, bei der die erfindungsgemäße Vorrichtung üblicherweise verwendet wird. Die genannte Maßnahme beschreibt eine weitere, alternative Möglichkeit, eine Vorrichtung mit dem erfindungsgemäßen Verbindungsglied zu realisieren.

In einer weiteren Ausgestaltung der zuvor genannten Maßnahmen ist das Verbindungsglied ein torusförmiger Körper, in dessen Öffnung das Haltemittel angeordnet ist.

Diese Maßnahme besitzt den Vorteil, daß das Verbindungsglied rund um das Haltemittel herum angeordnet ist und somit eine in allen Richtungen gleichmäßige Flexibilität bzw. Steifigkeit gewährleistet. Außerdem wird hierbei eine besonders hohe Stabilität der Vorrichtung in dem zweiten Betriebszustand erreicht.

In einer weiteren Ausgestaltung der zuvor genannten Maßnahmen ist das Basisteil an einem Tragegestell befestigbar, das seinerseits an einem Operationstisch einstellbar ortsfest montierbar ist.

Alternativ zu dieser Maßnahme ist es auch denkbar, die erfindungsgemäße Vorrichtung ausschließlich an dem Körper des Patienten zu fixieren, sei es allein durch ihr Gewicht oder zusätzlich durch die Verwendung eines wieder lösbaren Klebstoffs. Die genannte Maßnahme besitzt demgegenüber jedoch den Vorteil, daß das Basisteil und somit die gesamte Vorrichtung wesentlich stabiler und fester in bezug zu dem Patienten gehalten werden. Hierdurch ist gewährleistet, daß die Vorrichtung sich,auch bei Bewegungen des Patienten nicht unbeabsichtigt verschieben kann. Gleichzeitig ist durch die einstellbar ortsfeste Montierbarkeit an dem Operationstisch gewährleistet, daß die Vorrichtung insgesamt stets optimal in bezug zu dem Patienten positioniert werden kann.

In einer weiteren Ausgestaltung der zuvor genannten Maßnahme weist das Tragegestell zumindest eine Schiene auf, die den Operationstisch überspannt, und zwar vorzugsweise in etwa in Querrichtung bezogen auf den Patienten.

Diese Maßnahme besitzt den Vorteil, daß eine besonders gute Stabilität erreicht wird, ohne daß die Vorrichtung zuviel Raum einnimmt und dadurch den Zugang zu dem Patienten behindert. Außerdem kann eine derartige Schiene auf einfache Weise so angeordnet werden, daß das Basisteil der Vorrichtung praktisch direkt auf dem Körper des Patienten aufliegt, wodurch Relativbewegungen zwischen der Trokarhülse und dem Patienten weitgehend ausgeschlossen werden.

In einer weiteren Ausgestaltung der zuvor genannten Maßnahme ist das Basisteil verschiebbar an der Schiene befestigbar.

Diese·Maßnahme besitzt den Vorteil, daß die Haltevorrichtung schnell und auf einfache Weise verstellbar ist und von daher stets in eine optimale Position in bezug zu dem Patienten gebracht werden kann.

In einer weiteren Ausgestaltung der zuvor genannten Maßnahmen ist die zumindest eine Schiene entlang des Operationstisches verschiebbar und/oder um einen Schwenkpunkt verschwenkbar.

Auch diese Maßnahmen tragen dazu bei, die Einsatzmöglichkeiten und die Positionierbarkeit der erfindungsgemäßen Vorrichtung zu vergrößern. Gleichzeitig ist die Bedienung der Vorrichtung dabei sehr einfach, und die Vorrichtung stellt keine Behinderung beim Zugang zu dem Patienten dar. Darüber hinaus ist die Maßnahme im Vergleich zu variabel einstellbaren, mehrgliedrigen Gelenkarmen einfach und kostengünstig in der Konstruktion und Herstellung.

In einer weiteren Ausgestaltung der zuvor genannten Maßnahmen weist das Tragegestell mehrere Schienen auf, an denen mehrere Basisteile mit Haltemitteln und Verbindungsgliedern befestigbar sind.

Diese Maßnahme besitzt den Vorteil, daß durch die Verwendung mehrerer Schienen die Stabilität der Vorrichtung verbessert ist. Die Verwendung mehrerer Basisteile bietet darüber hinaus den Vorteil, daß mehrere medizinische Instrumente an verschiedenen Stellen des Patienten gleichzeitig fixierbar sind.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine teilweise geschnittene, perspektivische Darstellung einer Vorrichtung zum Halten einer Trokarhülse, von der die Beschreibung der Erfindung ausgeht,
- Fig. 2: einen Operationstisch mit mehreren Haltevorrichtungen von oben,
- Fig. 3: eine alternative Ausführung eines Operationstisches mit Haltevorrichtungen,
- Fig. 4: die Verwendung der Haltevorrichtung in einer Querschnittsdarstellung, wobei ein medizinisches Instrument in einer ersten Position fixiert ist,
- Fig. 5: die Verwendung der Haltevorrichtung entsprechend Fig. 4, wobei das medizinische Instrument in einer gegenüber der ersten Position geneigten, zweiten Position fixiert ist, und
- Fig. 6: ein zweites Ausführungsbeispiel einer Haltevorrichtungen in einer teilweise geschnittenen, perspektivischen Ansicht.

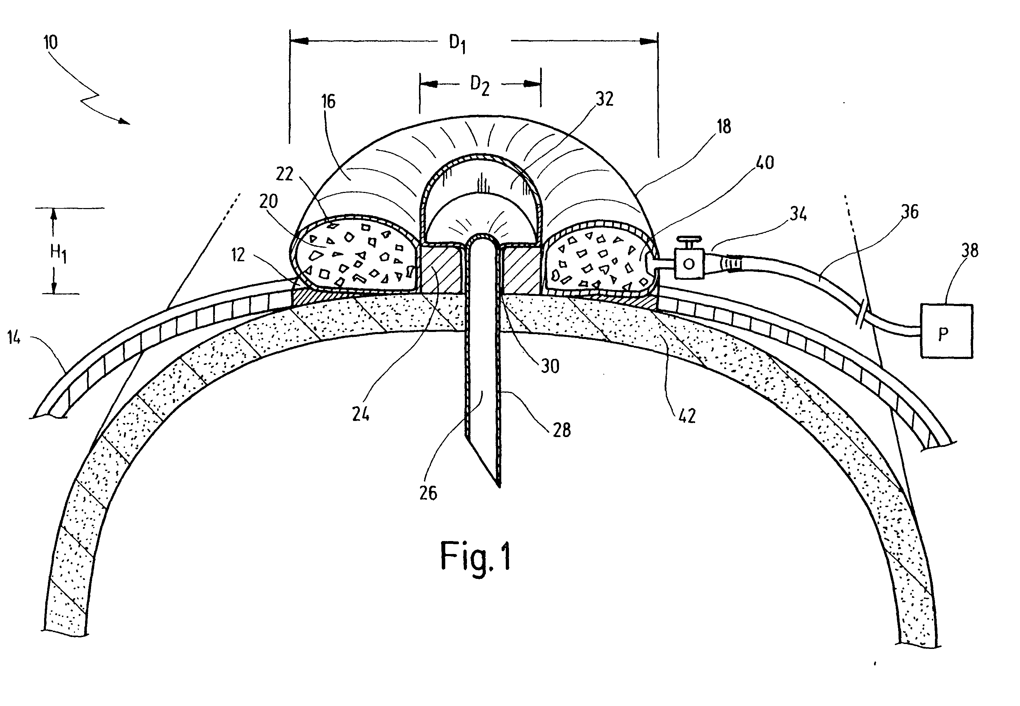

In Fig. 1 ist eine Haltevorrichtung in ihrer Gesamtheit mit der Bezugsziffer 10 bezeichnet.

Die Haltevorrichtung 10 weist ein Basisteil 12 auf, das an einer Schiene 14 eines hier nur ausschnittsweise dargestellten Tragegestells befestigt ist. Das Basisteil 12 ist im vorliegenden Fall ein Stützring, an dem ein torusförmiges Verbindungsglied 16 befestigt ist. Das Verbindungsglied 16 kann an dem Basisteil 12 angeklebt oder geschraubt sein. Im vorliegenden Beispiel ist das Verbindungsglied 16 jedoch an dem Basisteil 12 festgeklemmt und somit auch abnehmbar. In einer weiteren alternativen Ausführungsform kann das Basisteil 12 in das Verbindungsglied 16 integriert sein. In diesem Fall besteht das Basisteil 12 dann im wesentlichen aus einer versteiften Unterseite des Verbindungsgliedes 16.

Das Verbindungsglied 16 weist eine elastische Hülle 18 auf, die im vorliegenden Fall aus Silikon hergestellt ist. Die Hülle 18 ist hier mit einem partikelförmigen Material 20 gefüllt, dessen einzelne Partikel 22 eine Oberfläche mit im wesentlichen ebenen Flächenabschnitten und Kanten besitzen. Hierdurch verhaken sich die einzelnen Partikel 22 besonders gut aneinander, wenn sie zusammengepreßt werden. Eine solche Füllung der Hülle 18 fällt jedoch nicht unter die Ausführungsbeispiele der Erfindung. Die Beschreibung dient hier der vorbereitenden Erläuterung eines nachfolgend beschriebenen Ausführungsbeispiels.

Die torusförmige, mit den Partikeln 22 gefüllte Hülle 18 umgibt ein ebenfalls ringförmiges Haltemittel 24, an dessen äußeren Umfang sie angeklebt ist. Das Haltemittel 24 besteht aus einem im Vergleich zu der Hülle 18 festen Material, wie beispielsweise Kunststoff, und kann alternativ zu dem hier dargestellten Ausführungsbeispiel auch hohl ausgebildet sein oder Kanäle aufweisen.

Mit der Bezugsziffer 26 ist eine hier nur schematisch dargestellte Trokarhülse bezeichnet. Die Trokarhülse 26 besitzt einen rohrförmigen Schaft 28, der in der dargestellten Position durch die Ringöffnung 30 des Haltemittels 24 gesteckt ist und der sich an seinem oberen Ende zu einen ebenfalls hohlzylindrischen Ventilgehäuse 32 aufweitet.

Das Ventilgehäuse 32 der Trokarhülse 26 besitzt üblicherweise eine Ventilklappe zum Verschließen des Schaftes 28 und kann darüber hinaus auch eine Klemmvorrichtung und/oder einen oder mehrere Anschlüsse aufweisen. Derartige Ausführungen von Trokaren bzw. Trokarhülsen sind jedoch im Stand der Technik an sich bekannt und sind hier aus Gründen der Übersichtlichkeit nicht dargestellt.

Mit der Bezugsziffer 34 ist ein Be- und Entlüftungsventil bezeichnet, das über eine Leitung 36 mit einer Pumpe 38 verbunden ist. Das Ventil 34 mündet in der Hülle 18 des Verbindungsglieds 16 und besitzt ein Sperrsieb 40, dessen Gitteröffnung so bemessen ist, daß die Partikel 22 nicht aus der Hülle 18 austreten können.

Mit Hilfe des Ventils 34 sowie der Pumpe 38 ist es möglich, Luft oder ein beliebiges Gas in die Hülle 18 des Verbindungsglieds 16 hineinzupumpen bzw. aus dieser abzusaugen. Letzteres führt dazu, daß in der Hülle 18 ein Unterdruck erzeugt wird, durch den sich die Hülle 18 zusammenzieht. Die Folge davon ist, daß die einzelnen Partikel 22 des Materials 20 ihren Bewegungsspielraum verlieren und zusammengepreßt werden. Dies wiederum führt dazu, daß das ansonsten flexible Verbindungsglied 16 in seiner dann vorliegenden Form "fixiert wird". Da die dann steife Masse das Haltemittel 24 umgibt, hält die steife Masse dieses Haltemitel 24 in einer bestimmten Raumausrichtung. Durch Belüften der Hülle 18 wird demgegenüber die ursprüngliche Bewegungsfreiheit der Partikel 22 und somit die ursprüngliche Flexibilität des Verbindungsglieds 16 wieder hergestellt.

Mit der Bezugsziffer 42 ist die Bauchdecke eines zu behandelnden Patienten bezeichnet, durch die der Schaft 28 der Trokarhülse 26 eingestochen ist. Die Trokarhülse 26 ist somit um einen sogenannten invarianten Punkt, der in der Bauchdecke 42 liegt, verkippbar, sofern sich die Vorrichtung 10 in ihrem ersten flexiblen Betriebszustand befindet.

Die Abmessungen des Verbindungsglieds 16 sind in Fig. 1 mit den Größen D₁, D₂ und H₁ bezeichnet und betragen im vorliegenden Fall ungefähr D₁ = 100 mm, D₂ = 30 mm und H₁ = 20 mm.

Alternativ zu dem beschriebenen Fall, bei dem innerhalb der Hülle 18 ein partikelförmiges Material 20 verwendet ist und bei dem die Steifigkeit des Verbindungsglieds 16 mit Hilfe des Ventils 34 und der Pumpe 38 veränderbar ist, kann die Hülle 18 gemäß der vorliegenden Erfindung auch mit einer Flüssigkeit gefüllt sein, deren Viskosität zwischen weichelastisch und steif einstellbar ist. Bevorzugt ist die Flüssigkeit dabei mit Hilfe eines elektrischen und/oder eines magnetischen Signals einstellbar. Anstelle des Ventils 34 ist in diesem Fall dann ein hier nicht dargestellter elektrischer Anschluß bzw. eine magnetische Ankopplung sowie anstelle der Pumpe 38 ein entsprechender Feldgenerator erforderlich. Die Hülle 18 sowie ihre Verbindung mit dem Haltemittel 24 kann dabei jedoch in derselben Art und Weise realisiert sein, wie dies in Fig. 1 dargestellt ist.

Gleiches gilt auch für den Fall, daß als Material 20 ein Material verwendet wird, das bei einer ersten einstellbaren Betriebstemperatur flexibel und bei einer zweiten Betriebstemperatur steif ist. Anstelle des Ventils 34 und der Pumpe 38 werden in diesem Fall dann entsprechende Mittel benötigt, mit denen die Temperatur des verwendeten Materials möglichst schnell veränderbar ist. Dies besteht in einer kombinierten Heiz/Kühleinheit, und das Material ist so ausgewählt, daß es einen scharfen Schmelzpunkt im Bereich knapp über der Raumtemperatur aufweist.

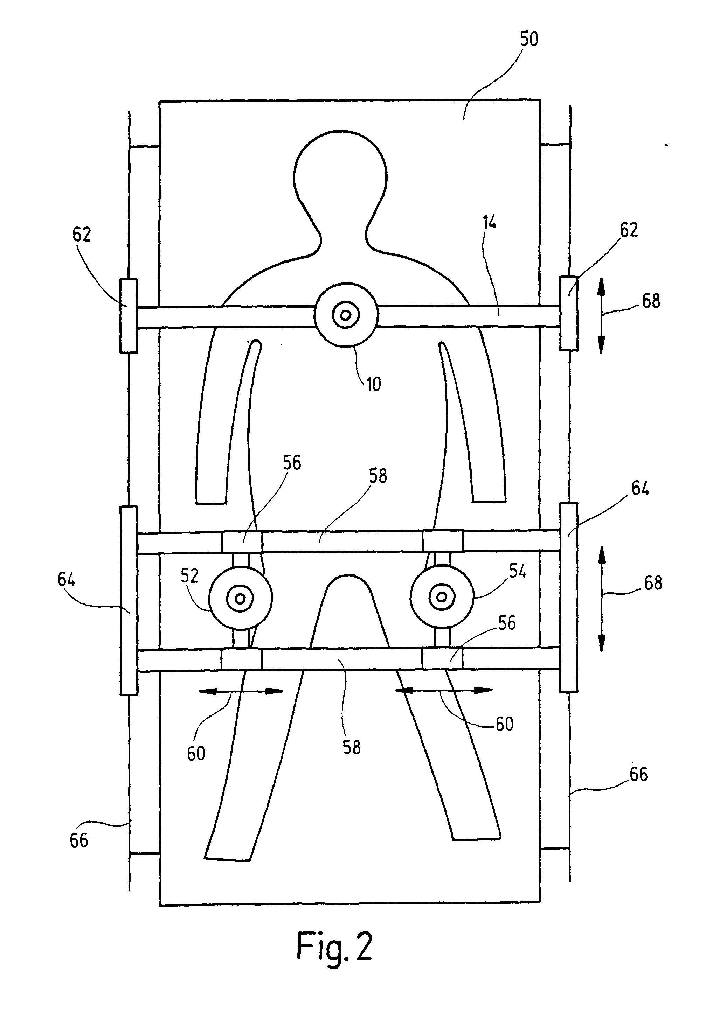

In Fig. 2 ist ein Operationstisch 50 beispielhaft mit insgesamt drei Haltevorrichtungen 10 bzw. 52 und 54 ausgerüstet. Es versteht sich, daß ein solcher Operationstisch jedoch auch mit nur einer oder auch mit jeder anderen Anzahl von Haltevorrichtungen 10 versehen sein kann.

Die Haltevorrichtung 10 ist mit ihrem Basisteil 12 an der Schiene 14 des bereits im Hinblick auf Fig. 1 erwähnten Tragegestells befestigt. Die Haltevorrichtungen 52 und 54 sind demgegenüber jeweils an einem Halter 56 befestigt, die auf zwei Schienen 58 in Richtung der Pfeile 60 verschieblich gelagert sind. Darüber hinaus ist es durch die Verschieblichkeit der Haltevorrichtungen 52, 54 in Richtung der Pfeile 60 möglich, die Vorrichtungen 52, 54 entlang der Breite des Operationstisches in eine gewünschte Position zu bringen.

Die Schiene 14 ist ebenso wie die beiden Schienen 58 über Laufelemente 62, 64 mit Laufschienen 66 gekoppelt, die beidseitig in Längsrichtung des Operationstisches 50 an diesem befestigt sind. Aufgrund der Laufelemente 62, 64 ist es möglich, die Schiene 14 bzw. die Anordnung aus den beiden Schienen 58 in Längsrichtung des Operationstisches 50, d.h. in Richtung der Pfeile 68, zu verschieben. Die Laufelemente 62, 64 können Rollen oder andere Gleitmittel aufweisen und darüber hinaus mit hier nicht dargestellten Feststellmitteln versehen sein.

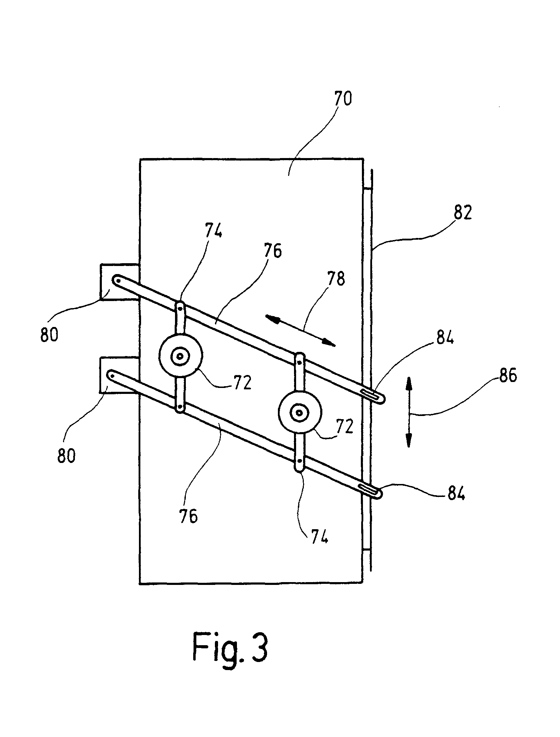

In Fig. 3 ist ein Operationstisch mit dem Bezugszeichen 70 bezeichnet. Die erfindungsgemäßen Haltevorrichtungen 72 sind auch hier wiederum an Haltern 74 befestigt, die an Schienen 76 in Richtung des Pfeils 78 verschieblich. gelagert sind. Im Unterschied zum vorhergehenden Ausführungsbeispiel sind die beiden Schienen 76 im vorliegenden Fall jedoch mit ihrem einen Ende auf Tellern 80 dreh- bzw. verschwenkbar gelagert. Das andere Ende der Schienen 76 ist demgegenüber über hier nicht näher dargestellte Stifte mit einer Laufschiene 82 verbunden. Dabei sind die Stifte jeweils in einem Langloch 84 an jeder Schiene 76 eingehängt, so daß ein Längenausgleich beim Verschwenken gegeben ist. Die Laufschiene 82 ist wiederum an einer Längsseite des Operationstisches 70 an diesem befestigt. Mit Hilfe dieser Anordnung ist es ebenfalls möglich, die erfindungsgemäßen Vorrichtungen 72 in Längsrichtung des Operationstisches 70, d.h. in Richtung des Pfeils 86, zu verstellen.

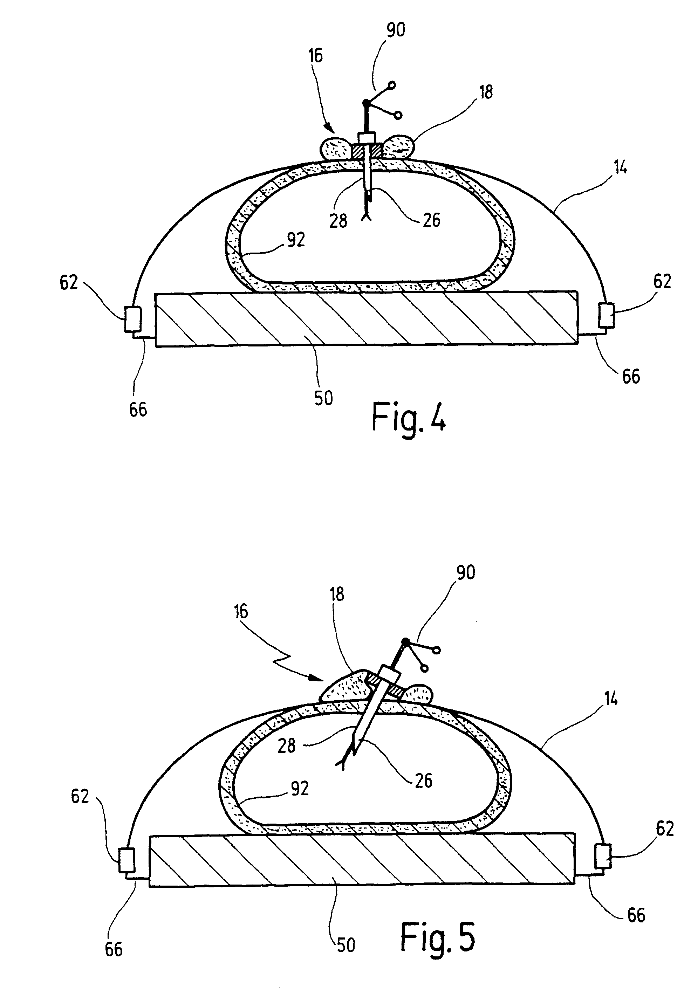

Nachfolgend wird anhand der Fig. 4 und 5 die Verwendung der Haltevorrichtung beschrieben. Gleiche Bezugszeichen bezeichnen dabei dieselben Elemente, die bereits anhand der vorhergehenden Figuren erläutert wurden.

Mit der Bezugsziffer 90 ist ein medizinisches Instrument bezeichnet, das durch die Trokarhülse 26 in den Körper 92 eines zu behandelnden Patienten eingeführt ist. Das medizinische Instrument ist im vorliegenden Beispiel eine Zange.

In Fig. 4 befindet sich die Trokarhülse 26 etwa in derselben Position, die auch in Fig. 1 gezeigt ist. Der Schaft 28 der Trokarhülse 26 steht dabei im wesentlichen senkrecht. Eine derartige Ausrichtung ist jedoch häufig nicht geeignet, eine gewünschte Stelle innerhalb des Körpers 92 des Patienten zu erreichen. Vielmehr muß das medizinische Instrument 90 häufig schräg durch den Einschnitt im Körper 92 des Patienten geführt werden, um ein bestimmtes Organ zu erreichen.

Dazu wird die Haltevorrichtung zu Beginn der Behandlung mit Hilfe der Schienen 14 bzw. 58, 76 in eine gewünschte Position relativ zu dem Patienten gebracht. Anschließend wird mit Hilfe eines Trokars der benötigte Einschnitt in dem Körper 92 des Patienten vorgenommen, wobei die Trokarhülse 26 dann im Körper 92 des Patienten stecken bleibt und in der gewünschten Richtung und Winkellage ausgerichtet werden kann. Letzteres kann beispielsweise unter Zuhilfenahme eines optischen Instrumentes geschehen, das in diesem Fall dann durch die Trokarhülse 26 hindurchgeführt wird.

Während der Positionssuche befindet sich das Verbindungsglied 16 in seinem ersten flexiblen Betriebszustand. Die Trokarhülse 26 kann in diesem Betriebszustand mitsamt dem Haltemittel 24 relativ zu dem Basisteil 12 verkippt werden, wodurch sich das torusförmigen Verbindungsglied 16 verformt.

Wenn die gefundene Position eingenommen ist, wird das Verbindungsglied 16 in seinen zweiten, steifen Betriebszustand versetzt, und seine zuletzt eingenommene Form wird fixiert. Gleichzeitig wird dabei auch das Haltemittel 24 und folglich die Trokarhülse 26 in der eingestellten Ausrichtung fixiert. Ein solcher Zustand ist beispielhaft in Fig. 5 dargestellt.

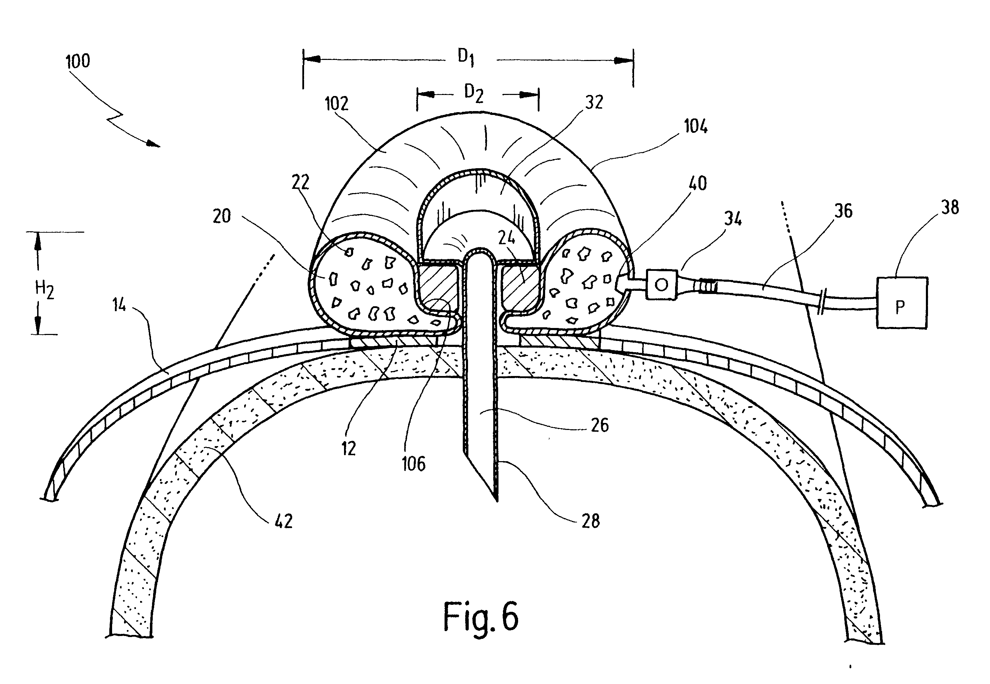

In Fig. 6 ist eine weitere Haltevorrichtung in ihrer Gesamtheit mit der Bezugsziffer 100 bezeichnet. Gleiche Bezugszeichen bezeichnen des weiteren wiederum Elemente, die bereits anhand der vorhergehenden Figuren erläutert wurden.

Die Vorrichtung 100 unterscheidet sich von der Vorrichtung 10 im wesentlichen dadurch, daß das Verbindungsglied 102 eine Hülle 104 aufweist, die bis unter das Haltemittel 24 reicht, so daß dieses nahezu vollständig in der Hülle 104 eingebettet ist. Darüber hinaus sind die Kanten 106 des Haltemittels 24 hier deutlich abgerundet ausgeführt, um eine Beschädigung der Hülle 104 beim Verstellen der Position der Trokarhülse 26 zu verhindern. Durch die Unterfütterung des Haltemittels 24 mit der Hülle 104 und dem in ihr enthaltenen Material 20 wird die Stabilität der Trokarhülse 26 vor allem dann verstärkt, wenn diese sehr stark in eine Richtung geneigt ist.

In einem weiteren, hier jedoch nicht dargestellten Ausführungsbeispiel kann das Haltemittel 24 in die Hülle 104 integriert sein. In diesem Fall liegt der Kopf 32 der Trokarhülse 26 dann direkt auf der Hülle 104 auf.

## Patentansprüche

1. Vorrichtung zum Halten einer Trokarhülse (26) in unterschiedlichen räumlichen Ausrichtungen relativ zu einem Patienten, mit einem Basisteil (12), das relativ zu dem Patienten ortsfest positionierbar ist, und mit einem Haltemittel (24) für die Trokarhülse (26), wobei das Haltemittel (24) mit dem Basisteil (12) durch ein flexibles Verbindungsglied (16; 102) verkippbar verbunden ist, wobei das Verbindungsglied (16; 102) einen ersten Betriebszustand aufweist, in dem es flexibel ist, sowie einen zweiten Betriebszustand, in dem es steif ist, und wobei das Verbindungsglied (16; 102) zwischen dem ersten flexiblen und dem zweiten steifen Betriebszustand umschaltbar ist, **dadurch gekennzeichnet, daß** das Verbindungsglied (16; 102) eine elastische Hülle (18; 104) aufweist, die mit einem Material (20) veränderbarer Steifigkeit gefüllt ist.

2. Vorrichtung nach Anspruch 1, **dadurch gekennzeichnet, daß** das Material (20) eine Flüssigkeit ist, deren Viskosität zwischen weichelastisch und steif einstellbar ist.

3. Vorrichtung nach Anspruch 2, **dadurch gekennzeichnet, daß** die Viskosität des Materials (20) unter dem Einfluß eines elektrischen Signals einstellbar ist.

4. Vorrichtung nach Anspruch 2 oder 3, **dadurch gekennzeichnet, daß** die Viskosität des Materials (20) unter dem Einfluß eines magnetischen Signals einstellbar ist.

5. Vorrichtung nach Anspruch 1, **dadurch gekennzeichnet, daß** das Verbindungsglied (16; 102) ein Material (20) aufweist, das bei einer einstellbaren ersten Betriebstemperatur flexibel und bei einer zweiten Betriebstemperatur steif ist.

6. Vorrichtung nach einem der Ansprüche 1 bis 5, **dadurch gekennzeichnet, daß** das Verbindungsglied (16; 102) ein torusförmiger Körper ist, in dessen Öffnung das Haltemittel (24) angeordnet ist.

7. Vorrichtung nach einem der Ansprüche 1 bis 6, **dadurch gekennzeichnet, daß** sie ein Tragegestell beinhaltet, das an einem Operationstisch (50; 70) einstellbar ortsfest montierbar ist, wobei das Basisteil (12) an dem Tragegestell befestigbar ist.

8. Vorrichtung nach Anspruch 7, **dadurch gekennzeichnet, daß** das Tragegestell zumindest eine Schiene (14; 58; 76) aufweist, die den Operationstisch (50; 70) überspannt, und zwar vorzugsweise in etwa in Querrichtung bezogen auf den Patienten.

9. Vorrichtung nach Anspruch 8, **dadurch gekennzeichnet, daß** das Basisteil (12) verschiebbar an der Schiene (14; 58; 76) befestigbar ist.

10. Vorrichtung nach Anspruch 8 oder 9, **dadurch gekennzeichnet, daß** die zumindest eine Schiene (14; 58; 76) entlang des Operationstisches (50; 70) verschiebbar ist

11. Vorrichtung nach einem der Ansprüche 8 bis 10, **dadurch gekennzeichnet, daß** die zumindest eine Schiene (14; 58; 76) an dem Operationstisch (50; 70) um einen Schwenkpunkt (80) verschwenkbar gelagert ist.

12. Vorrichtung nach einem der Ansprüche 8 bis 11, **dadurch gekennzeichnet, daß** das Tragegestell mehrere Schienen (58; 76) aufweist, an denen mehrere Basisteile (12; 52; 54; 72) mit Haltemitteln (24) und Verbindungsgliedern (16; 102) befestigbar sind.

## Claims

1. Apparatus for holding a trocar sleeve (26) in different spatial alignments relative to a patient, comprising a base (12) positionable to be fixed relative to the patient and a retainer (24) for the trocar sleeve (26), where the retainer (24) is connected to be tiltable with respect to the base (12) by a flexible coupling member (16; 102), wherein the coupling member (16; 102) has a first operational state in which it is flexible and a second operational state in which it is rigid, and wherein the coupling member (16; 102) can be switched between the first flexible and the second rigid operational state, **characterized in that** the coupling member (16; 102) comprises an elastic casing (18; 104) filled with a material (20) being variable in rigidity.

2. Apparatus according to claim 1, **characterized in that** the material (20) is a fluid whose viscosity is adjustable between being elastic and rigid.

3. Apparatus according to claim 2, **characterized in that** the viscosity of the material (20) is adjustable through the influence of an electric signal.

4. Apparatus according to claim 2 or 3, **characterized in that** the viscosity of the material (20) is adjustable through the influence of a magnetic signal.

5. Apparatus according to claim 1, **characterized in that** the coupling member (16; 102) comprises a material which is flexible at an adjustable first operational temperature and is rigid at a second operational temperature.

6. Apparatus according to any one of the claims 1 to 5, **characterized in that** the coupling member (16; 102) is a torus-shaped body where the retainer (24) is arranged in its opening.

7. Apparatus according to any one of the claims 1 to 6, **characterized by** a support frame, which can be adjustably and fixedly mounted to an operating table (50; 70), with the base (12) being securable to the support frame.

8. Apparatus according to claim 7, **characterized in that** the support frame comprises at least one rail (14; 58; 76) arranged over the operating table (50; 70), preferably in approximately transverse direction with respect to the patient.

9. Apparatus according to claim 8, **characterized in that** the base (12) is slidably securable to the rail (14; 58; 76).

10. Apparatus according to claim 8 or 9, **characterized in that** the at least one rail (14; 58; 76) is shiftable along the operating table (50; 70).

11. Apparatus according to any one of the claims 8 to 10, **characterized in that** the at least one rail (14; 58; 76) is mounted to the operating table (50; 70) to be pivotal about a pivot point (80).

12. Apparatus according to any one of the claims 8 to 10, **characterized in that** the support frame comprises several rails (58; 76) to which several bases (12; 52; 54; 72) with retainers (24) and coupling members (16; 102) are securable.

## Revendications

1. Dispositif pour le maintien d'un manchon de trocart (26) selon des orientations spatiales différentes par rapport à un patient, avec une partie de base (12), qui peut être positionnée fixement par rapport au patient, et avec un moyen de retenue (24) pour le manchon de trocart (26), le moyen de retenue (24) étant relié à la partie de base (12) de manière à pouvoir basculer à l'aide d'un élément de liaison souple (16 ; 102), l'élément de liaison (16 ; 102) présentant un premier état de fonctionnement dans lequel il est souple et un second état de fonctionnement dans lequel il est rigide, et l'élément de liaison (16 ; 102) pouvant être commuté entre le premier état de fonctionnement souple et le second état de fonctionnement rigide, **caractérisé en ce que** l'élément de liaison (16 ; 102) comporte un manchon élastique (18 ; 104) qui est rempli d'une matière (20) ayant une rigidité variable.

2. Dispositif selon la revendication 1, **caractérisé en ce que** la matière (20) est un liquide dont la viscosité est réglable entre souple et élastique et rigide.

3. Dispositif selon la revendication 2, **caractérisé en ce que** la viscosité de la matière (20) est réglable sous l'influence d'un signal électrique.

4. Dispositif selon la revendication 2 ou 3, **caractérisé en ce que** la viscosité de la matière (20) est réglable sous l'influence d'un signal magnétique.

5. Dispositif selon la revendication 1, **caractérisé en ce que** l'élément de liaison (16 ; 102) comporte une matière (20) qui est souple à une première température de fonctionnement réglable et qui est rigide à une seconde température de fonctionnement.

6. Dispositif selon l'une quelconque des revendications 1 à 5, **caractérisé en ce que** l'élément de liaison (16 ; 102) est un corps en forme de tore dans l'ouverture duquel est placé le moyen de retenue (24).

7. Dispositif selon l'une quelconque des revendications 1 à 6, **caractérisé en ce qu'**il comprend un cadre porteur qui peut être monté fixement de manière réglable sur une table d'opération (50 ; 70), la partie de base (12) pouvant être fixée au cadre porteur.

8. Dispositif selon la revendication 7, **caractérisé en ce que** le cadre porteur comprend au moins un rail (14 ; 58 ; 76) qui enjambe la table d'opération (50 ; 70) et ce, de préférence, à peu près dans le sens transversal par rapport au patient.

9. Dispositif selon la revendication 8, **caractérisé en ce que** la partie de base peut être fixée de manière coulissante au rail (14 ; 58 ; 76).

10. Dispositif selon la revendication 8 ou 9, **caractérisé en ce que** le au moins un rail (14 ; 58 ; 76) peut coulisser le long de la table d'opération (50 ; 70).

11. Dispositif selon l'une quelconque des revendications 8 à 10, **caractérisé en ce que** le au moins un rail (14 ; 58 ; 76) est logé sur la table d'opération (50 ; 70) de manière à pouvoir pivoter autour d'un point de pivotement (80).

12. Dispositif selon l'une quelconque des revendications 8 à 11, **caractérisé en ce que** le cadre porteur comprend plusieurs rails (58 ; 76) auxquels plusieurs parties de base (12 ; 52 ; 54 ; 72) avec des moyens de retenue (24) et des éléments de liaison (16 ; 102) peuvent être fixées.
